# FASCICULE DE BREVET EUROPEEN

(11) **EP 1 493 421 B1**
(45) Date de publication et mention de la délivrance du brevet: **14.03.2007**
(21) Numéro de dépôt: 04291414.3
(22) Date de dépôt: 07.06.2004
(51) Int. Cl.: A61Q 1/00, A61Q 19/00, A61K 8/04, A61K 8/19, A61K 8/25, A61K 8/26, A61K 8/29, A61K 8/34, A61K 8/67, A61K 8/73, A61K 8/60, A61K 8/81, A61K 8/891, A61K 8/892, A61K 8/90, A61K 8/895

(54) **Composition pour application topique contenant un organopolysiloxane élastomère et une dispersion aqueuse de copolymère siliconé bloc**
Topische Zusammensetzung enthaltend ein Organopolysiloxan-Elastomer, sowie eine wässrige Dispersion eines Silikon-Block-Copolymers
Topical composition containing an organopolysiloxane elastomer, and an aqueous dispersion of silicon bloc copolymer

(30) Priorité: 02.07.2003 FR 0308028
(43) Date de publication de la demande: 05.01.2005
(73) Titulaire: L'ORÉAL, 75008 Paris (FR)
(72) Inventeur: Lebreton, Francoise, 91440 Bures-sur-Yvette (FR); Pierre, Patricia, 92160 Antony (FR)
(74) Mandataire: Rasson, Catherine

(56) Documents cités:
- EP-A- 1 016 400
- EP-A- 1 095 959
- US-A- 5 266 321
- US-A- 5 412 004
- US-A- 5 928 660
- US-A- 6 074 672
- US-A1- 2002 018 790

## Description

La présente demande se rapporte à une composition pour application topique contenant un organopolysiloxane solide élastomère, une charge, un alcool primaire et une dispersion aqueuse d'un copolymère siliconé bloc, et aux utilisations de la dite composition, notamment dans le domaine cosmétique, en particulier pour matifier la peau et pour estomper les défauts du relief de la peau tels que les microreliefs, les rides, les pores tout en conférant à la peau un aspect naturel.

Les produits dits « perfecteurs de peau » deviennent de plus en plus recherchés par les consommatrices. Il s'agit de compositions de soin de la peau ou de maquillage ayant des propriétés matifiantes, généralement utilisées pour résoudre les problèmes de brillance occasionnés par un excès de sébum et pour améliorer la tenue du maquillage à long terme qui a tendance à se dégrader visuellement au cours de la journée. Ces compositions sont particulièrement recherchées pour les utilisateurs à peaux mixtes ou grasses, ainsi que sous les climats chauds et humides. Ces compositions matifiantes donnent à la peau un aspect mat résultant d'un pouvoir diffusant de la lumière à la surface de la peau ; c'est ce que l'on appelle l'effet « soft focus ». Une composition présentant des propriétés de « soft-focus » est telle que, lorsqu'on l'applique sur la peau, elle donne un effet flou qui camoufle les microreliefs et autres imperfections de la peau.

On connaît par le document EP-A-790055 des compositions matifiantes contenant l'association d'un organopolysiloxane solide élastomère avec une phase grasse, conférant à la peau un aspect mat de façon prolongée dans le temps.

EP 1016 400 propose des compositions à usage externe pour lisser la peau en conférant un aspect naturel.

Pour apporter un bon effet matifiant, l'organopolysiloxane est souvent utilisé à des taux importants, par exemple au moins 2 % en poids de matière active. En outre, il est souvent associé à des charges pour améliorer les effets optiques et/ou sensorielles de la composition. Toutefois, la composition obtenue donne alors des produits qui, lors de l'application sur la peau, ont un effet trop sec et donnent des dépôts trop poudrés. L'ajout d'huiles émollientes et d'agents hydratants à haut poids moléculaire apporte des propriétés hydratantes et émollientes accrues. Néanmoins, cet ajout s'accompagne d'une lourdeur et d'une épaisseur de la texture, et donc occasionne une perte des premières qualités recherchées qui sont la légèreté et l'effet évanescent.

Il subsiste donc le besoin d'une composition matifiante ayant un bon effet "soft focus" tout en étant agréable à utiliser, c'est-à-dire légère, et sans effet sec ni gras à l'application sur la peau.

La demanderesse a trouvé de manière surprenante que l'incorporation de particules d'un copolymère siliconé bloc substantiellement linéaire en dispersion aqueuse et d'un alcool primaire aux compositions contenant un organopolysiloxane élastomère et des charges apportait des propriétés hydratantes et émollientes surprenantes, associées à des textures légères, veloutées et évanescentes.

La composition ainsi obtenue présente l'avantage d'être à la fois légère et filmogène, de s'étaler parfaitement sur la peau sans avoir ni effet collant ni effet gras, et d'apporter un effet émollient. La peau sur laquelle a été appliquée la composition est souple, élastique et matte, l'effet optique de la composition permettant en outre de voiler les imperfections cutanées.

Ainsi, la présente l'invention a pour objet une composition pour application topique, comprenant au moins un organopolysiloxane solide élastomère, au moins une charge, au moins un alcool primaire en C₂-C₃ et une dispersion aqueuse d'au moins un copolymère siliconé bloc substantiellement linéaire.

La composition de l'invention étant destinée notamment à une application topique, elle comprend un milieu physiologiquement acceptable, c'est-à-dire un milieu compatible avec toutes les matières kératiniques telles que la peau, les ongles, les muqueuses et les cheveux ou toute autre zone cutanée du corps. Elle peut constituer notamment une composition cosmétique ou dermatologique.

### Copolymère siliconé bloc substantiellement linéaire

Le copolymère siliconé constituant les globules ou particules en dispersion dans la phase aqueuse est un copolymère bloc substantiellement linéaire, c'est-à-dire un copolymère non réticulé, obtenu par extension de chaîne et non par réticulation, par réaction d'extension de chaîne, en présence d'un catalyseur, à partir d'au moins :
- (a) un polysiloxane (i) ayant au moins un groupe réactif et de préférence un ou deux groupes réactifs par molécule ; et
- (b) un composé organosiliconé (ii) qui réagit avec le polysiloxane (i) par réaction d'extension de chaîne.

La dispersion aqueuse de particules de copolymère bloc est une émulsion silicone dans eau (Sil/E), dont les globules huileux sont constitués d'un silicone de viscosité élevée, de sorte que ces globules semblent former comme "des particules souples".

La taille des particules de copolymère siliconé bloc substantiellement linéaire peut varier largement. De manière préférée dans la présente demande, les particules de copolymère siliconé bloc substantiellement linéaire présentent généralement une taille moyenne en nombre inférieure ou égale à 2 microns, et de préférence inférieure ou égale à 1 micron.

Les dispersions aqueuses de particules de copolymères siliconés blocs substantiellement linéaires, utilisées dans la composition selon l'invention peuvent être choisies notamment parmi celles décrites dans le document EP-A-874017.

En particulier, le polysiloxane (i) est choisi parmi les composés de formule (I) : 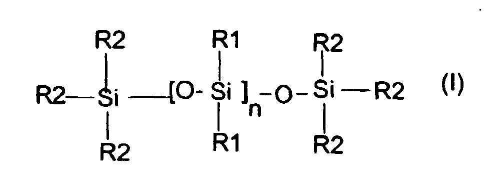 dans laquelle R₁ et R₂ indépendamment les uns des autres représentent un groupe hydrocarboné ayant de 1 à 20 atomes de carbone et de préférence de 1 à 10 atomes de carbone, tel que méthyle, éthyle, propyle ou butyle, ou un groupe aryle tel que phényle, ou un groupe réactif, n est un nombre entier supérieur à 1, à la condition qu'il y ait en moyenne entre un et deux groupes réactifs par polymère.

On entend par « groupe réactif » tout groupe susceptible de réagir avec le composé organosiliconé (ii) pour former un copolymère bloc. Comme groupes réactifs, on peut citer l'hydrogène ; les groupes aliphatiquement insaturés et notamment vinyle, allyle ou hexanyle ; le groupe hydroxyle ; les groupes alcoxy tels que méthoxy, éthoxy ou propoxy ; les groupes alcoxy-alcoxy ; le groupe acétoxy ; les groupes aminés, et leurs mélanges. De préférence, plus de 90 % et mieux plus de 98% de groupes réactifs sont en bout de chaîne, c'est-à-dire que les radicaux R₂ constituent généralement plus de 90 % et même 98% des groupes réactifs.

De préférence, n est tel que les polysiloxanes ont une viscosité allant d'environ 1 à 1.10⁶ mm²/sec à 25°C. n peut être notamment un nombre entier allant de 5 à 30, de préférence de 10 à 30 et mieux de 15 à 25.

Les polysiloxanes de formule (I) sont des polymères substantiellement linéaires, c'est-à-dire comportant peu de ramifications, et généralement moins de 2 % en mole des unités siloxane. Par ailleurs, les groupes R₁ et R₂ peuvent être éventuellement substitués par des groupes aminés, des groupes époxy, des groupes comportant du soufre, du silicium ou de l'oxygène.

De préférence, au moins 80 % des groupes R₁ sont des groupes alkyle et mieux des groupes méthyle.

De préférence, le groupe réactif R₂ en bout de chaîne est un groupe aliphatiquement insaturé et notamment vinyle.

Comme polysiloxanes (i), on peut citer notamment le diméthylvinyl-siloxy-polydiméthylsiloxane, composé de formule (I) dans laquelle les radicaux R₁ sont des radicaux méthyle, et, les radicaux R₂ en bout de chaîne sont des radicaux vinyle tandis que les deux autres radicaux R₂ sont des radicaux méthyle.

Le composé organosiliconé (ii) peut être choisi parmi les polysiloxanes de formule (I) ou les composés agissant comme agent d'extension de chaîne. Si c'est un composé de formule (I), le polysiloxane (i) comportera un premier groupe réactif et le composé organosiliconé (ii) comportera un second groupe réactif qui réagira avec le premier. Si c'est un agent d'extension de chaîne, ce peut être un silane, un siloxane (disiloxane ou trisiloxane) ou un silazane. De préférence, le composé organosiliconé (ii) est un organohydrogenopolysiloxane liquide de formule (II) : 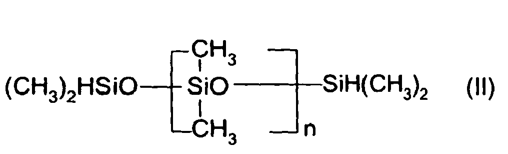 où n est un nombre entier supérieur à 1 et de préférence supérieur à 10, et par exemple allant de 5 à 30, de préférence de 10 à 30 et mieux de 15 à 25. Selon un mode particulier de réalisation de l'invention, n est égal à 20.

Les copolymères blocs siliconés utilisés selon l'invention sont exempts de groupe oxyalkyléné, notamment exempts de groupes oxyéthyléné et/ou oxypropyléné.

Le catalyseur de la réaction entre le polysiloxane et le composé organosiliconé peut être choisi parmi les métaux et notamment parmi le platine, le rhodium, l'étain, le titane, le cuivre et le plomb. Il s'agit de préférence du platine ou du rhodium.

La dispersion de particules de copolymère siliconé utilisée dans la composition selon l'invention peut être notamment obtenue par exemple par mélange de (a) l'eau, (b) au moins un émulsifiant, (c) le polysiloxane (i), (d) le composé organosiliconé (ii) et (e) un catalyseur. De préférence, l'un des constituants (c), (d) ou (e) est ajouté en dernier dans le mélange, afin que la réaction d'extension de chaîne ne commence que dans la dispersion.

Comme émulsifiants susceptibles d'être utilisés dans le procédé de préparation décrit ci-dessus pour obtenir la dispersion aqueuse de particules, on peut citer les émulsifiants non ioniques ou ioniques (anioniques, cationiques ou amphotères). Il s'agit de préférence d'émulsifiants non ioniques qui peuvent être choisis parmi les polyalkylène glycol éthers d'alcool gras, comportant de 8 à 30 atomes de carbone et de préférence de 10 à 22 atomes de carbone ; les alkylesters de sorbitan polyoxyalkylénés et notamment polyoxyéthylénés, où le radical alkyle comporte de 8 à 30 atomes de carbone et de préférence de 10 à 22 atomes de carbone ; les alkylesters polyoxyalkylénés et notamment polyoxyéthylénés, où le radical alkyle comporte de 8 à 30 atomes de carbone et de préférence de 10 à 22 atomes de carbone ; les polyéthylène glycols ; les polypropylène glycols ; les diéthylène glycols ; et leurs mélanges. La quantité d'émulsifiant(s) est généralement de 1 à 30 % en poids par rapport au poids total du mélange de réaction.

L'émulsifiant utilisé pour obtenir la dispersion aqueuse de particules est de préférence choisi parmi les polyéthylène glycol éthers d'alcools gras et leurs mélanges, et notamment les polyéthylène glycol éthers d'alcools comportant 12 ou 13 atomes de carbone et de 2 à 100 motifs oxyéthylénés et de préférence de 3 à 50 motifs oxyéthylénés, et leurs mélanges. On peut citer par exemple le C₁₂-C₁₃ Pareth-3, le C₁₂-C₁₃ Pareth-23 et leurs mélanges.

Selon un mode particulier de réalisation de l'invention, la dispersion de particules de copolymère siliconé est obtenue à partir de diméthylvinyl-siloxy-polydiméthylsiloxane (ou divinyldimethicone) comme composé (i), et du composé de formule (II) avec de préférence n=20, comme composé (ii), de préférence en présence d'un catalyseur de type platine, et la dispersion de particules est de préférence obtenue en présence de C₁₂-C₁₃ Pareth-3 et C₁₂-C₁₃ Pareth-23 comme émulsifiants.

Comme dispersion de particules de copolymère siliconé, on peut utiliser notamment le produit commercialisé sous la dénomination HMW 2220 par la société Dow Corning (nom CTFA : divinyldimethicone/dimethicone Copolymer / C₁₂-C₁₃ Pareth-3 / C₁₂-C₁₃ Pareth-23), qui est une dispersion aqueuse à 60 % de copolymère divinyldimethicone / dimethicone, contenant du C₁₂-C₁₃ Pareth-3 et du C₁₂-C₁₃ Pareth-23, la dite dispersion comprenant environ 60 % en poids de copolymère ; 2,8 % en poids de C₁₂-C₁₃ Pareth-23 ; 2 % en poids de C₁₂-C₁₃ Pareth-3 ; 031 % en poids de conservateurs, le reste à 100 % étant de l'eau.

La quantité de particules de copolymère siliconé bloc substantiellement linéaire peut aller par exemple de 0,01 à 15 % en poids de matière active de copolymère, de préférence de 0,05 à 10 % en poids, et mieux de 0,05 à 5 % en poids par rapport au poids total de la composition, ce qui représente par exemple une quantité de HMW 2220 de 0,01 à 25 % en poids, de préférence de 0,08 à 17 % en poids et mieux de 0,08 à 8,3 % en poids par rapport au poids total de la composition puisque cette matière première contient 60 % en poids de copolymère.

### Organopolysiloxane élastomère

La composition de l'invention contient au moins un organopolysiloxane élastomère, de préférence au moins partiellement réticulé. On entend par « élastomère » un matériau solide souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomères utilisés dans la composition selon l'invention sont de préférence partiellement ou totalement réticulés. Ils se présentent sous forme de particules. En particulier, les particules d'organopolysiloxane élastomère ont une taille allant de 0,1 à 500 µm, de préférence de 3 à 200 µm et mieux de 3 à 50 µm. Ces particules peuvent avoir toute forme et par exemple être sphériques, plates ou amorphes.

Quand ils sont inclus dans une phase huileuse, ces organopolysiloxanes élastomères se transforment, selon le taux de phase huileuse utilisé, en un produit d'aspect spongieux lorsqu'ils sont utilisés en présence de faibles teneurs en phase huileuse, ou en un gel homogène en présence de quantités de phase huileuse plus élevées. La gélification de la phase huileuse par ces élastomères peut être totale ou partielle.

Ainsi les élastomères de l'invention peuvent être véhiculés sous forme de gel anhydre constitué d'un organopolysiloxane élastomère et d'une phase huileuse. La phase huileuse utilisée lors de la fabrication du gel anhydre d'organopolysiloxane élastomère contient une ou plusieurs huiles liquides à températures ambiante (25°C) choisies parmi les huiles hydrocarbonées et/ou les huiles de silicone. Avantageusement, la phase huileuse est une phase liquide siliconée, contenant une ou plusieurs huiles choisies parmi les polydiméthylsiloxanes à chaîne linéaire ou cyclique, liquides à température ambiante comportant éventuellement une chaîne alkyle ou aryle pendante ou en bout de chaîne, la chaîne alkyle ayant de 1 à 6 atomes de carbone.

Les organopolysiloxanes élastomères utilisés selon l'invention peuvent être choisis parmi les polymères réticulés décrits dans la demande EP-A-0295886 et parmi ceux décrits dans le brevet US-A-5,266,321.

Ce sont de préférence des organopolysiloxanes élastomères obtenus par réaction d'addition et de réticulation, en présence d'un catalyseur, de préférence un catalyseur du type platine, d'au moins :
- (a) un organopolysiloxane (iii) ayant deux groupements vinyliques en position α-ω de la chaîne siliconée par molécule; et
- (b) un organopolysiloxane (iv) ayant au moins deux atomes d'hydrogène liés à un atome de silicium par molécule.

Le premier organopolysiloxane (iii) est choisi parmi les polydiméthylsiloxanes ; il s'agit de préférence d' un α-ω-diméthylvinyl polydiméthylsiloxane.

L'organopolysiloxane est sous forme d'un gel obtenu selon les étapes suivantes :
- (a) mélange du premier et second organopolysiloxanes (iii) et (iv) ;
- (b) ajout d'une phase huileuse au mélange de l'étape (a) ;
- (c) polymérisation du premier et second organopolysiloxanes (iii) et (iv) en phase huileuse en présence d'un catalyseur, de préférence d'un catalyseur de platine.

Les organopolysiloxanes élastomères utilisés dans la composition de l'invention peuvent être par exemple ceux commercialisés sous les noms KSG 6 par la société Shin-Etsu ; Trefil E-505C ou Trefil E-506C par la société Dow-Corning ; Gransil (SR-CYC, SR DMF10, SR-DC556) par la société Grant Industries, ou ceux commercialisés sous forme de gels déjà constitués : KSG 15, KSG 16, KSG 17, KSG 18, KSG 26A, KSG 26B, de la société Shin-Etsu ; Gransil SR 5CYC gel, Gransil SR DMF 10 gel, Gransil SR DC556 gel de la société Grant Industries ; 1229-02-167 et 1229-02-168 de la société General Electric. On peut aussi utiliser un mélange d'élastomères de silicone, et notamment un mélange de ces produits commerciaux.

De manière préférée, l'organopolysiloxane élastomère utilisé dans la composition de l'invention se présente sous forme d'un gel anhydre, et notamment d'un gel anhydre formé de particules non sphériques d'organopolysiloxane élastomère, tels que les KSG. L'organopolysiloxane élastomère est préférentiellement introduit dans la phase huileuse de l'émulsion selon l'invention.

Le ou les organopolysiloxanes élastomères utilisés selon l'invention sont de préférence présents en une quantité en matière active allant de 2 à 20 % en poids, et mieux de 2 à 10 % en poids en poids par rapport au poids total de la composition.

### Charge

On entend par "charge" tout solide finement divisé. Elle peut être choisie parmi les charges organiques, les charges minérales et leurs mélanges.

Selon les cas, la charge peut être introduite dans la composition après mélange des autres constituants et, par exemple, dans le cas d'une émulsion après préparation de l'émulsion, ou bien, si une phase huileuse est présente, dans la phase huileuse de la composition. Elle peut aussi être introduite pendant la préparation de l'émulsion, dans la phase aqueuse ou dans la phase huileuse.

### Charge organique

Comme charges organiques (appelées aussi poudres organiques) pouvant être utilisées dans la composition de l'invention, on peut citer par exemple les particules de polyamide et notamment la poudre de NYLON 12, comme les produits commercialisés sous les dénominations ORGASOL par la société Atochem ; les poudres et billes de polyéthylène telles que celles commercialisées sous les dénominations ACUMIST (Acumist B-6, Acumist B-12) par la société Allied et celles commercialisées sous les dénominations MICROTHENE par la société Equistar ; les microsphères à base de copolymères acryliques ou méthacryliques, telles que celles en copolymère diméthacrylate d'éthylène glycol/methacrylate de lauryle, vendues par la société Dow Corning sous la dénomination de POLYTRAP, ou celles en copolymère méthacrylate de méthyle / diméthacrylate d'éthylène glycol, commercialisées sous la dénomination Microsphères M305 par la société Matsumoto ; les microsphères de polyméthacrylate de méthyle commercialisées sous la dénomination MICROSPHERE M100 par la société Matsumoto ou sous les dénominations COVABEAD (par exemple Covabead LH 85 ayant une dimension de 10 à 12 microns) par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous les dénominations TOSPEARL (par exemple Tospearl 2000 B ayant une dimension de 6 microns environ) par la société Toshiba Silicone ; et leurs mélanges.

### Charges minérales

Les charges minérales utilisées dans la composition de l'invention peuvent être très variées. Elles peuvent être choisies notamment parmi le talc ; le kaolin ; le nitrure de bore ; les oxydes métalliques ; les micas ; les nacres ; les poudres de silice (ou dioxyde de silicium) ; l'oxychlorure de bismuth ; le stéarate de zinc ; les particules de sel de métal alcalin ou alcalinoterreux comme les particules de carbonate de calcium, de sulfate de baryum, de sulfate de calcium ; les particules de platine ; les alumines (notamment les alumines actives) ; les alumino-silicates (argiles notamment) ; les silicates mixtes de métaux alcalins et/ou alcalino-terreux (smectites, Laponites, en particulier LAPONITES DS, D, XLS ou XLG commercialisées par la société Laporte Industries, Ltd., Saponites, en particulier VEEGUM commercialisé par la société Vanderbilt ; les zéolithes ; la magnésie ; les matériaux composites à base de charges minérales ; et leurs mélanges.

Comme oxydes métalliques, on peut citer par exemple les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique ayant une taille moyenne de particules allant par exemple de 5 à 100 nm. Ces oxydes métalliques peuvent être enrobés et notamment avoir un enrobage hydrophobe. Les oxydes métalliques enrobés pouvant être utilisés dans la composition de l'invention peuvent par exemple avoir subi un ou plusieurs traitements avec un ou plusieurs composés choisis parmi l'alumine, la silice, les dérivés de l'aluminium (par exemple stéarate et laurate), les composés du silicium (par exemple silicones, polydiméthylsiloxanes, alcoxysilanes, siloxy-silicates), les composés du sodium, les oxydes de fer, les esters de fer (par exemple stéarate), les acides gras, les alcools gras et leurs dérivés (tels que l'acide stéarique, l'acide hydroxystéarique, l'alcool stéarylique, l'alcool hydroxystéarylique, l'acide laurique et leurs dérivés), la lécithine, les cires (par exemple la cire de carnauba), les polymères (méth)acryliques (par exemple les polyméthylmethacrylates), les composés fluorés (par exemple les composés perfluoroalkyle et les perfluoroalkyle éthers). Les oxydes peuvent aussi être traités par un mélange de ces composés ou ils peuvent comprendre plusieurs enrobages successifs.

On peut aussi utiliser des nacres. Par nacres, il faut comprendre des particules irisées qui réfléchissent la lumière. Parmi les nacres envisageables, on peut citer la nacre naturelle, le mica recouvert d'oxyde de titane, d'oxyde de fer, d'hydroxyde d'aluminium, d'hydroxyde de magnésium, de silice, de pigment naturel et d'oxychlorure de bismuth, ainsi que le mica titane coloré, et leurs mélanges.

La silice pouvant être utilisée comme charge minérale peut être choisie parmi les microsphères de silice, comme celles commercialisées sous la dénomination SUNSPHERE H-51 par la société Asahi Glass, ou sous la dénomination Silica Beads par la Société MAPRECOS.

On peut aussi utiliser les silices hydrophiles ou hydrophobes commercialisées par la société Degussa-Hüls sous les dénominations AEROSIL 90, AEROSIL 130, AEROSIL 150, AEROSIL 200, AEROSIL300, AEROSIL380, AEROSIL OX 50, SILICE FK 320 DS, AEROSIL R202, AEROSIL R805, AEROSIL R812, AEROSIL R972, AEROSIL R974.

On peut encore utiliser de la silice en dispersion aqueuse, et par exemple une dispersion de silice colloïdale, telle que le produit commercialisé sous la dénomination BINDZIL 30/220® par la société Eka Chemicals, dispersion colloïdale de silice amorphe (taille : 14 nanomètres) dans l'eau (30/70), ou le produit commercialisé sous la dénomination COSMO S-40 par la société Catalysts Chemicals (taille : 18 nanomètres).

La silice peut consister également en une particule recouverte totalement ou partiellement de silice, notamment d'une particule minérale recouverte totalement ou partiellement de silice, telle que les billes de silice contenant de l'oxyde de titane, commercialisées sous la dénomination TORAYCERAM S-IT® par la société Toray ; les microsphères de silice-alumine contenant de l'oxyde de titane (taille : 105 µm), commercialisées sous la dénomination Z-LIGHT-SPHERE W 1012® par la société Zeelan ; les particules de silice synthétique précipitée amorphe/oxyde de titane (taille : 106-500 µm), commercialisées sous la dénomination NEOSIL PC20S® par la société Crosfield ; les fibres de Nylon-6 - silice - oxyde de titane (longueur de 2 mm et épaisseur de 2 deniers), commercialisées sous la dénomination FIBERLON Y2® par la société Wackherr ; la silice enrobée de dioxyde de titane et recouvert de silice poreuse (85/5/10) (taille : 0,6 µm), commercialisée sous la dénomination ACS-0050510® par la société SACI-CFPA ; le nano-oxyde de titane anatase traité alumine et silice à 40% dans l'eau (taille : 60 nm, monodisperse), commercialisé sous la dénomination MIRASUN TIW 60® par la société Rhodia Chimie CRA ; le nano-oxyde de titane anatase (60 nm) enrobé silice/alumine/cerium IV 15/5/3 en dispersion aqueuse à 32 %, commercialisé sous la dénomination MIRASUN TIW 160® par la société Rhodia Chimie CRA ; le nano-oxyde de titane anatase traité alumine et silice (34/4,3/1,7) en dispersion aqueuse à 40 %, commercialisé sous la dénomination TIOVEIL AQ-N® par la société Uniqema ; le nano-oxyde de titane enrobé de silice (66/33) (granulométrie du dioxyde de titane : 30 nm ; épaisseur de silice : 4 nm), commercialisé sous la dénomination MAXLIGHT TS-04® par la société Nichimen Europe PLC ; et le nano-oxyde de titane enrobé de silice (80/20) (granulométrie dioxyde de titane : 30 nm ; épaisseur de silice : 2 nm) commercialisé sous la dénomination MAXLIGHT TS-042® par la société Nichimen Europe PLC. Ces particules peuvent également avoir des propriétés optiques dans la composition comme sur la peau.

La ou les charges (organiques et/ou minérales) utilisées dans la composition conforme à l'invention sont généralement présentes dans des quantités en matière active allant de 0,01 à 30 % en poids, plus préférentiellement de 0,05 à 20 %, encore plus préférentiellement de 0,1 à 10 % en poids par rapport au poids total de la composition.

### Alcool primaire en C₂-C₃

On entend par "alcool primaire en C₂-C₃" un mono-alcool inférieur aliphatique, linéaire ou ramifié ayant de 2 à 3 atomes de carbone. Selon un mode préféré de réalisation de l'invention, l'alcool primaire utilisé est l'éthanol.

La quantité en alcool inférieur peut aller par exemple de 2 à 20 % en poids, de préférence de 3 à 10 % en poids par rapport au poids total de la composition.

Les compositions selon l'invention peuvent se présenter sous toutes les formes galéniques classiquement utilisées pour une application topique et notamment sous forme de gels, de dispersions, d'émulsions de consistance liquide ou semi-liquide du type lait, obtenues par dispersion d'une phase grasse (appelée aussi phase huileuse) dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un mode préféré de réalisation de l'invention, la composition se présente sous forme d'une émulsion et notamment d'une émulsion H/E.

La présente invention a aussi pour objet une composition pour application topique sous forme d'émulsion H/E, contenant une phase huileuse dispersée dans une phase aqueuse, la dite composition contenant au moins une charge, la phase huileuse contenant au moins un organopolysiloxane solide élastomère, la phase aqueuse contenant au moins un alcool primaire en C₂-C₃ et une dispersion aqueuse d'au moins un copolymère siliconé bloc substantiellement linéaire.

En outre, les compositions utilisées selon l'invention peuvent être plus ou moins fluides et avoir par exemple l'aspect d'un gel ou d'une crème blanche ou colorée. Elles peuvent être éventuellement appliquées sur la peau sous forme d'aérosol. Elles peuvent aussi se présenter sous forme solide, et par exemple sous forme de poudre, sous forme coulée, en coupelle ou sous forme de stick. Elles se présentent de préférence sous forme d'une crème fluide.

La composition de l'invention comprend une phase aqueuse qui peut n'être constituée que de la dispersion aqueuse des particules de copolymères siliconés blocs et de l'alcool mais qui comprend généralement d'autres constituants hydrosolubles ou hydrodispersibles. Dans les compositions substantiellement anhydres, c'est-à-dire comportant moins de 5 % en poids d'eau par rapport au poids totale de la composition, la phase aqueuse n'est constituée que de la dispersion aqueuse des particules de copolymères siliconés blocs et de l'alcool primaire. Selon la forme galénique de la composition, la quantité de phase aqueuse peut aller de 0,1 à 99 % en poids, de préférence de 0,5 à 98 % en poids, mieux de 30 à 95 % en poids, et encore mieux de 40 à 95 % en poids par rapport au poids total de la composition. Cette quantité dépend de la forme galénique de la composition désirée. La quantité d'eau peut représenter tout ou une partie de la phase aqueuse, et elle est généralement d'au moins 30 % en poids par rapport au poids total de la composition, notamment quand la composition se présente sous forme d'émulsion.

Les compositions de l'invention peuvent contenir dans la phase aqueuse ou dans la phase huileuse si elle comporte une phase huileuse, outre l'alcool primaire, un ou plusieurs solvants organiques, hydrophiles, lipophiles et/ou amphiphiles, physiologiquement acceptables, c'est-à-dire bien tolérés et donnant un toucher cosmétiquement acceptable.

Les solvants organiques peuvent représenter de 0,5 à 50 % et de préférence de 2 à 20 % du poids total de la composition. Les solvants organiques peuvent être choisis dans le groupe constitué par les solvants organiques hydrophiles, les solvants organiques lipophiles, les solvants amphiphiles, ou leurs mélanges.

Parmi les solvants organiques autres que l'alcool primaire, on peut citer par exemple les polyols tels que le propylène glycol, l'isoprène glycol, le butylène glycol, le propylène glycol, le glycérol, le sorbitol ; les mono- ou di-alkyle isosorbide dont les groupements alkyle ont de 1 à 5 atomes de carbone comme le diméthyl isosorbide ; les polyéthylène glycols notamment ceux ayant de 6 à 80 oxydes d'éthylène, tels que le polyéthylène glycol 32 OE ; les éthers d'éthylène glycol comme le diéthylène glycol mono-méthyl ou mono-éthyl éther ; les éthers de propylène glycol comme le dipropylène glycol méthyl éther ; les esters et éthers de polyol, tels que les esters de polypropylène glycol (PPG) et plus spécialement les esters de polypropylène glycol (PPG) et d'acide gras, les éthers de PPG et d'alcool gras comme le PPG-23 oléyl éther et le PPG-36 oléate ; les esters d'acide gras et d'alkyle, tels que l'adipate de diisopropyle, l'adipate de dioctyle, les benzoates d'alkyle ; et leurs mélanges.

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Pour les émulsions E/H, on peut citer par exemple comme émulsionnants les dimethicone copolyols tels que le mélange de cyclomethicone et de dimethicone copolyol, vendu sous la dénomination DC 5225 C par la société Dow Corning ou celui vendu sous la dénomination ABIL EM 97 par la société Goldschmidt, et les alkyl-dimethicone copolyols tels que le Laurylmethicone copolyol vendu sous la dénomination Dow Corning 5200 Formulation Aid par la société Dow Corning et le Cetyl dimethicone copolyol vendu sous la dénomination ABIL EM 90 par la société Goldschmidt, ou le mélange polyglycéryl-4 isostéarate/cétyl diméthicone copolyol/hexyllaurate vendu sous la dénomination ABIL WE 09 par la société Goldschmidt. On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénés et/ou oxypropylénés, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

Comme ester de glycéryle et d'acide gras, on peut citer notamment le stéarate de glycéryle (mono-, di- et/ou tri-stéarate de glycéryle) (nom CTFA : glyceryl stearate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stearate), le monostéarate de polyéthylène glycol 100 OE (nom CTFA : PEG-100 stearate et leurs mélanges.

On peut aussi utiliser des mélanges de ces tensioactifs, comme par exemple le produit contenant du Glyceryl stearate et du PEG-100 stearate, commercialisé sous la dénomination ARLACEL 165 par la société Uniqema, et le produit contenant du Glyceryl stearate (mono-distéarate de glycéryle) et du stéarate de potassium, commercialisé sous la dénomination TEGIN par la société Goldschmidt (nom CTFA : glyceryl stearate SE).

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate) ; l'ester de méthylglucoside et d'acide isostéarique (nom CTFA : Methyl glucose isostearate) ; l'ester de méthylglucoside et d'acide laurique (nom CTFA : Methyl glucose laurate) ; le mélange de monoester et de diester de méthylglucoside et d'acide isostéarique (nom CTFA : Methyl glucose sesquiisostearate) ; le mélange de monoester et de diester de méthylglucoside et d'acide stéarique (nom CTFA : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société Amerchol ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société Amerchol et celui commercialisé sous la dénomination Grillocose PSE-20 par la société Goldschmidt, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le decylglucoside comme le produit commercialisé sous la dénomination MYDOL 10 par la société Kao Chemicals, le produit commercialisé sous la dénomination PLANTAREN 2000 par la société Cognis, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Cognis ; le coco-glucoside comme le produit commercialisé sous la dénomination PLANTACARE 818/UP par la société Cognis ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Cognis ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic ; et leurs mélanges.

Quand la composition se présente sous forme d'une émulsion, la nature de la phase huileuse de l'émulsion n'est pas critique. La phase huileuse peut ainsi être constituée par tous les corps gras et notamment les huiles, classiquement utilisés dans les domaines cosmétique ou dermatologique. La phase huileuse comprend généralement au moins une huile, notamment une huile cosmétique.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, de coriandre, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R^{a}COOR^{b} et R^{a}OR^{b} dans laquelle R^{a} représente le reste d'un acide gras comportant de 8 à 29 atomes de carbone, et R^{b} représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures substantiellement linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12, le ceteareth-12 et le ceteareth-20 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluorométhoxybutane vendu sous la dénomination "MSX 4518®" par la Société 3M et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée substantiellement linéaire ou cyclique, liquides ou pâteux à température ambiante, notamment les cyclopolydiméthylsiloxanes (cyclométhicones) telles que la cyclohexadiméthylsiloxane et la cyclopentadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; la pâte de vaseline ; les gommes telles que les gommes de silicone (diméthiconol) ; les résines de silicone telles que la trifluorométhyl-C1-4-alkyldimethicone et la trifluoropropyldimethicone.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition de l'invention contient au moins une huile de silicone, de préférence une huile de silicone volatile qui peut être choisie par exemple parmi les polydiméthylsiloxanes cycliques ou substantiellement linéaires, et leurs mélanges. Les polydiméthylsiloxanes cycliques ou cyclométhicones comportent d'environ 3 à 9 atomes de silicium, et de préférence de 4 à 6 atomes de silicium et peuvent être par exemple le cyclohexadiméthyl-siloxane et le cyclopentadiméthylsiloxane. Les polydiméthylsiloxanes substantiellement linéaires volatiles comportent de préférence d'environ 3 à 9 atomes de silicium. Les polydiméthylsiloxanes substantiellement linéaires volatiles ont généralement une viscosité à 25°C inférieure ou égale à 5 cSt tandis que les cyclométhicones ont généralement une viscosité à 25°C inférieure ou égale à 10 cSt.

De façon connue, toutes les compositions de l'invention peuvent contenir un ou plusieurs des adjuvants habituels dans les domaines cosmétique et dermatologique, des agents gélifiants et/ou épaississants hydrophiles ou lipophiles ; des actifs hydrophiles ou lipophiles ; des séquestrants ; des antioxydants ; des conservateurs ; des agents alcanisants ou acidifiants ; des parfums ; des agents filmogènes ; des matières colorantes ; et leurs mélanges.

Les quantités de ces différents adjuvants sont celles classiquement utilisées dans les domaines considérés. En particulier, les quantités d'actifs varient selon le but recherché et sont celles classiquement utilisées dans les domaines considérés, et par exemple de 0,1 à 20 %, et de préférence de 0,5 à 10 % du poids total de la composition.

Comme gélifiants hydrophiles autres que les polymères décrits ci-dessus, on peut citer par exemple les polymères carboxyvinyliques tels que les carbopols (carbomers) et les Pemulen (Copolymère acrylate/C₁₀-C₃₀-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les dérivés cellulosiques tels que l'hydroxyéthylcellulose ; les polysaccharides et notamment les gommes telles que la gomme de xanthane ; et leurs mélanges.

Comme gélifiants lipophiles, on peut citer les argiles modifiées telles que le l'hectorite et ses dérivés, comme les produits commercialisés sous les noms de Bentone.

### Actifs

Comme actifs utilisables dans la composition de l'invention, on peut citer par exemple les agents hydratants tels que les hydrolysats de protéines, le hyaluronate de sodium, les polyols comme la glycérine, les glycols comme les polyéthylène glycols et les dérivés de sucre ; les extraits naturels ; des émollients ; des agents anti-radicaux libres ; les anti-inflammatoires ; les oligomères procyannidoliques ; les vitamines comme la vitamine A (rétinol), la vitamine E (tocophérol), la vitamine C (acide ascorbique), la vitamine B5 (panthénol), la vitamine B3 (niacinamide), les dérivés de ces vitamines, notamment leurs esters, et en particulier les dérivés de vitamine C tels que les esters d'ose de l'acide ascorbique comme l'ascorbyl-2 glucoside ou 2-O-α-D glucopyranosyl de l'acide L-ascorbique, et les sels métalliques d'acide ascorbique phosphorylé comme l'ascorbyl phosphate de magnésium ; l'urée ; la caféine ; les dépigmentants tels que l'acide kojique, l'hydroquinone et l'acide caféique ; l'acide salicylique et ses dérivés ; les alpha-hydroxyacides tels que l'acide lactique, l'acide glycolique et l'acide gluconique, et leurs dérivés, notamment leurs esters et aussi leurs sels tels que les gluconates, notamment les gluconates de magnésium, de manganèse, de calcium et de zinc ; les rétinoïdes tels que les caroténoïdes comme l'α-carotène, le β-carotène, la zéaxanthine, la cryptoxanthine, la lutéine et le lycopène ; les filtres solaires ; l'hydrocortisone ; la mélatonine ; les extraits d'algues, de champignons, de végétaux (par exemple extraits d'Ulmaire), de levures, de bactéries ; les enzymes ; les stéroïdes (par exemple la déhydroépiandrostérone (ou DHEA), ainsi que ses précurseurs et dérivés biologiques et chimiques, et/ou les extraits naturels en contenant et notamment les extraits de Dioscorées, tels que l'igname sauvage (Wild Yam) ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les agents matifiants comme les fibres ; les agents tenseurs ; et les mélanges de ces actifs.

Comme actif, la composition peut comprendre notamment un agent tenseur. Par "agent tenseur", on entend un composé capable d'exercer une traction sur la peau, qui a pour effet d'estomper temporairement les irrégularités de la surface de la peau, telles que les rides et ridules. Parmi les agents tenseurs utilisables dans la composition selon la présente invention, on peut citer notamment :
(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80 (nom INCI : Polysilicone 8), VS 70 ou LO21,
(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
(3) les protéines et hydrolysats de protéines végétales, en particulier de maïs, de seigle, de froment, de sarrasin, de sésame, d'épautre, de pois, de fève, de lentille, de soja et de lupin,
(4) les microparticules de cire, choisies par exemple parmi les cires de Carnauba, de Candelila ou d'Alfa,
(5) les particules colloïdales de charge inorganique ayant un diamètre moyen en nombre compris entre 0,1 et 100 nm, de préférence entre 3 et 30 nm, et choisies par exemple parmi : la silice, l'oxyde de cérium, l'oxyde de zirconium, l'alumine, le carbonate de calcium, le sulfate de baryum, le sulfate de calcium, l'oxyde de zinc et le dioxyde de titane.

Les filtres U.V. ou agents photoprotecteurs pouvant être utilisés dans la composition conforme à l'invention peuvent être choisis parmi les agents photoprotecteurs organiques et/ou au moins les agents photoprotecteurs inorganiques, actifs dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les documents US-A-4367390, EP-A-863145, EP-A-517104, EP-A-570838, EP-A-796851, EP-A-775698, EP-A-878469, EP-A-933376, EP-A-507691, EP-A-507692, EP-A-790243, EP-A-944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les documents EP-A-669323 et US-A-2,463,264 ; les dérivés de l'acide p-aminobenzoïque (PABA) ; les dérivés de méthylène bis-(hydroxyphényl benzotriazole) tels que décrits dans les documents US-A-5,237,071, US-A-5,166,355, GB-A-2303549, DE-A-197 26 184 et EP-A-893119 ; les polymères filtres et silicones filtres tels que ceux décrits notamment dans le document WO-A-93/04665 ; les dimères dérivés d'α-alkylstyrène tels que ceux décrits dans le document DE-A-19855649.

Comme exemples d'agents photoprotecteurs actifs dans l'UV-A et/ou l'UV-B, on peut citer plus particulièrement, ceux désignés ci-dessous sous leur nom INCI :
- les dérivés de l'acide para-aminobenzoïique, dont les suivants : PABA, Ethyl PABA, Ethyl Dihydroxypropyl PABA, Ethylhexyl Diméthyl PABA vendu notamment sous le nom ESCALOL 507 par ISP, Glyceryl PABA, PEG-25 PABA vendu sous le nom UVINUL P25 par BASF,
- les dérivés salicyliques, dont les suivants : Homosalate vendu notamment sous le nom NEO HELIOPAN OS par Haarmann et Reimer, Dipropyleneglycol Salicylate vendu notamment sous le nom DIPSAL par Scher, TEA Salicylate vendu notamment sous le nom NEO HELIOPAN TS par Haarmann et Reimer,
- les dérivés du dibenzoylméthane, dont les suivants : Butyl Methoxydibenzoylmethane vendu notamment sous le nom commercial PARSOL 1789 par Hoffmann Laroche, et Isopropyl dibenzoylmethane,
- les dérivés cinnamiques, dont les suivants : Ethylhexyl Methoxycinnamate vendu notamment sous le nom commercial PARSOL MCX par Hoffmann Laroche, Isopropyl Methoxycinnamate, Isoamyl Methoxycinnamate vendu notamment sous le nom commercial NEO HELIOPAN E 1000 par Haarmann et Reimer, Cinoxate, DEA Methoxycinnamate, Diisopropyl Methylcinnamate, Glyceryl Ethylhexanoate Dimethoxycinnamate,
- les dérivés de β,β-diphénylacrylate, dont les suivants : Octocrylene vendu notamment sous le nom commercial UVINUL N539 par BASF, Etocrylene vendu notamment sous le nom commercial UVINUL N35 par BASF,
- les dérivés de la benzophénone, dont les suivants : Benzophenone-1 vendue notamment sous le nom commercial UVINUL 400 par BASF, Benzophenone-2 vendue notamment sous le nom commercial UVINUL D50 par BASF, Benzophenone-3 ou Oxybenzone vendue notamment sous le nom commercial UVINUL M40 par BASF, Benzophenone-4 vendue notamment sous le nom commercial UVINUL MS40 par BASF, Benzophenone-5, Benzophenone-6 vendue notamment sous le nom commercial HELISORB 11 par Norquay, Benzophenone-8 vendue notamment sous le nom commercial SPECTRA-SORB UV-24 PAR American Cyanamid, Benzophenone-9 vendue notamment sous le nom commercial UVINUL DS-49 par BASF, Benzophenone-12, et le 2-(4-diéthylamino-2-hydroxybenzoyl)-benzoate de n-hexyle ;
- les dérivés du benzylidène camphre, dont les suivants : 3-Benzylidene camphor, 4-Methylbenzylidene camphor vendu notamment sous le nom EUSOLEX 6300 par Merck , Benzylidene Camphor Sulfonic Acid, Camphor Benzalkonium Methosulfate, Terephthalylidene Dicamphor Sulfonic Acid (Mexoryl SX de la société Chimex), Polyacrylamidomethyl Benzylidene Camphor,
- les dérivés de benzimidazole, dont les suivants : Phenylbenzimidazole Sulfonic Acid vendu notamment sous le nom commercial EUSOLEX 232 par Merck, Disodium Phenyl Dibenzimidazole Tetra-sulfonate vendu notamment sous le nom commercial NEO HELIOPAN AP par Haarmann et Reimer,
- les dérivés de triazine, dont les suivants : Anisotriazine vendu notamment sous le nom commercial TINOSORB S par Ciba Specialty Chemicals, Ethylhexyl triazone vendu notamment sous le nom commercial UVINUL T150 par BASF, Diethylhexyl Butamido Triazone vendu notamment sous le nom commercial UVASORB HEB par Sigma 3V, et la 2,4,6- tris-(4'amino-benzalmalonate de diisobutyle)-s-triazine,
- les dérivés de benzotriazole, dont les suivants : Drometrizole Trisiloxane vendu sous le nom SILATRIZOLE par Rhodia Chimie, Methylène bis-Benzotriazolyl Tetramethylbutylphénol, vendu notamment sous forme solide sous le nom commercial MIXXIM BB/100 par Fairmount Chemical ou sous forme micronisée en dispersion aqueuse sous le nom commercial TINOSORB M par Ciba Specialty Chemicals,
- les dérivés anthranilique, dont le Menthyl anthranilate vendu sous le nom commercial NEO HELIOPAN MA par Haarmann et Reimer,
- les dérivés d'imidazolines, dont l'Ethylhexyl Dimethoxybenzylidene Dioxoimidazoline Propionate,
- les dérivés de benzalmalonate, dont le polyorganosiloxane à fonctions benzalmalonate vendu sous la dénomination commerciale PARSOL SLX par Hoffmann Laroche,
- et leurs mélanges.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Méthylène bis-Benzotriazolyl Tetramethylbutylphénol, Drometrizole Trisiloxane, et leurs mélanges.

Les agents photoprotecteurs inorganiques peuvent être choisis parmi des pigments ou bien encore des nanopigments (taille moyenne des particules primaires: généralement entre 5 nm et 100 nm, de préférence entre 10 nm et 50 nm) d'oxydes métalliques, enrobés ou non enrobés, comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-518772 et EP-A-518773. On peut citer par exemple le nanotitane hydrophile commercialisé sous la dénomination MIRASUN TIW60 par la société Rhodia, et le nanotitane lipophile commercialisé sous la dénomination MT100T par la société TAYCA ou UV Titan M 160 par la société Kemira.

Les filtres U.V. quand ils sont présents dans les compositions selon l'invention, le sont généralement dans des proportions allant de 0,1 à 20 % en poids par rapport au poids total de la composition, et de préférence allant de 0,2 à 15 % en poids par rapport au poids total de la composition.

La quantité d'actifs dépend du but recherché. Le ou les actifs peuvent être par exemple présents d'une manière générale en une concentration allant de 0,001 à 20 %, de préférence de 0,01 à 10 % en poids et mieux de 0,05 à 5 % du poids total de la composition.

Selon un mode préféré de réalisation de l'invention, la composition comprend au moins un actif choisi parmi les agents tenseurs tels que les polymères siliconés greffés et notamment le Polysilicone 8 ; les sels d'alpha-hydroxyacides tels que les gluconates d'alpha-hydroxyacides ; les caroténoïdes et notamment le lycopène ; les dérivés de vitamine C et notamment l'ascorbyl-2 glucoside et l'ascorbyl phosphate de magnésium ; le hyaluronate de sodium ; les extraits végétaux et notamment l'extrait d'Ulmaire ; et leurs mélanges.

Bien entendu, l'homme de l'art veillera à choisir le ou les éventuels composés à ajouter à la composition selon l'invention et leurs quantités, de manière telle que les propriétés avantageuses attachées intrinsèquement à la composition conforme à l'invention ne soient pas ou substantiellement pas, altérées par l'addition envisagée.

La composition présente de préférence un pH qui respecte la peau et qui va généralement de 3 à 8 et de préférence de 4,5 à 7.

La composition selon l'invention peut constituer notamment une composition cosmétique, et en particulier un produit de soin, de protection et /ou de maquillage de la peau, notamment en vue de donner un aspect mat à la peau, et plus particulièrement pour estomper les défauts du relief de la peau tels que les microreliefs, les rides, les pores tout en conférant à celle-ci un aspect naturel.

Ainsi, l'invention a encore pour objet l'utilisation cosmétique d'une composition cosmétique telle que définie ci-dessus pour donner un aspect mat à la peau et/ou pour estomper les défauts du relief de la peau tels que les microreliefs, les rides, les pores tout en conférant à celle-ci un aspect naturel.

L'invention a aussi pour objet un procédé cosmétique pour donner un aspect mat à la peau et/ou pour estomper les défauts du relief de la peau, consistant à appliquer sur la peau, une composition cosmétique telle que définie ci-dessus.

Les exemples qui suivent servent à illustrer l'invention. Les quantités y sont données en pourcentages en poids.

### Exemple 1 : Emulsion HIE

| *Phase A (phase aqueuse)* | |
|---|---|
| Glycérine | 0,5 % |
| Gluconate de manganèse | 0,05 % |
| Ascorbyl glucoside | 0,5 % |
| Hyaluronate de sodium | 0,1 % |
| Ammonium polyacryldimethyl tauramide (Hostacerin AMPS) Acrylamide / sodium acryloyldimethyltaurate copolymer / | 1 % |
| Isohexadecane / polysorbate 80 (Simulgel 600) | 0,4 % |
| Magnesium Aluminium Silicate (Veegum) | 0,3 % |
| Conservateur | qs % |
| Eau | qsp 100 % |
| | |

| *Phase B (phase huileuse)* | |
|---|---|
| Organopolysiloxane élastomère (KSG 16 à 24 % de matière active) (soit 3,6 % de matière active) | 15 % |
| Cyclohexadiméthylsiloxane | 9 % |
| Acétate de tocophérol | 0,1 % |
| Polymethylsilsesquioxane (Tospearl 2000 B) | 0,5 % |
| | |

| *Phase C* | |
|---|---|
| Ethanol dénaturé | 10 % |
| | |

| *Phase D* | |
|---|---|
| Parfum | qs |
| | |

| *Phase E* | |
|---|---|
| Polymethyl methacrylate (Covabead LH 85) | 0,25 % |
| Mica / Oxyde de titane (56/44) | 0,5 % |
| | |

| *Phase F* | |
|---|---|
| HMW2220 (Dow Corning) | 2 % |

Mode opératoire : On prépare la phase A en chauffant à 80°C environ, le mélange d'eau, glycérine, conservateur, hyaluronate de sodium, ascorbyl glucoside et gluconate de manganèse, et en y dispersant les autres matières premières de la phase A (Hostacerin AMPS, Simulgel 600 et Veegum). Puis on y ajoute sous agitation la phase B préalablement chauffée à 60°C. Après refroidissement à température ambiante, on ajoute au mélange successivement les phases D, E et F sous agitation.

On obtient un fluide léger, nacré et très doux, ayant un bon effet matifiant.

### Test :

On a préparé une composition analogue à celle de l'exemple 1 mais ne contenant pas de phase F (exemple comparatif), et on a fait tester les deux compositions par dix personnes. Les personnes ont appliqué les compositions en demi visage (chaque composition sur une moitié du visage) et elles ont noté les sensations perçues lors de l'application du produit (douceur, émollience, caractère filmogène, effet sec). Les mêmes questions leur ont été posées après séchage du produit (environ deux minutes après application).

Ce test a montré que les 10 personnes ont toutes noté que la composition de l'exemple 1 selon l'invention avait un effet émollient et filmogène nettement amélioré par rapport à la composition de l'exemple comparatif, et que l'effet doux de la composition contenant KSG et charges était bien maintenu.

### Exemple 2 : Emulsion H/E

| *Phase A (phase aqueuse)* | |
|---|---|
| Glycérine | 1 % |
| Extrait d'ulmaire (Spiraea Ulmaria) | 0,1 % |
| Ammonium polyacryldimethyl tauramide (Hostacerin AMPS) | 0,7 % |
| Acrylamide / sodium acryloyldimethyltaurate copolymer / | |
| Isohexadecane / polysorbate 80 (Simulgel 600) | 0,2 % |
| Magnesium Aluminium Silicate (Veegum) | 0,3 % |
| Conservateur | qs % |
| Eau | qsp 100 % |
| | |

| *Phase B (phase huileuse)* | |
|---|---|
| Organopolysiloxane élastomère (KSG 16 à 24 % de matière active) (soit 3,6 % de matière active) | 15 % |
| Cyclohexadiméthylsiloxane | 9 % |
| Polysilicone 8 | 1 % |
| Dimethiconol (gomme de silicone) | 0,3 % |
| | |

| *Phase C* | |
|---|---|
| Ethanol dénaturé | 5 % |
| | |

| *Phase D* | |
|---|---|
| Parfum | qs |
| | |

| *Phase E* | |
|---|---|
| Polymethyl methacrylate (Covabead LH 85) | 0,25 % |
| Mica recouvert d'hydroxyde d'aluminium (65/35) | 0,25 % |
| | |

| *Phase F* | |
|---|---|
| HMW2220 (Dow Corning) | 2 % |

Le mode opératoire est analogue à celui de l'exemple 1. On obtient un fluide doux à l'application et émollient.

## Revendications

1. Composition pour application topique, comprenant au moins un organopolysiloxane solide élastomère, au moins une charge, au moins un alcool primaire en C₂-C₃ et une dispersion aqueuse d'au moins un copolymère siliconé bloc substantiellement linéaire non réticulé et exempt de groupe oxyalkyléné, obtenu par réaction d'extension de chaîne, en présence d'un catalyseur, à partir d'au moins :
- (a) un polysiloxane (i) ayant au moins un groupe réactif et de préférence un ou deux groupes réactifs par molécule ; et
- (b) un composé organosiliconé (ii) qui réagit avec le polysiloxane (i) par réaction d'extension de chaîne.

2. Composition selon la revendication 1, **caractérisée en ce que** la quantité de copolymère siliconé bloc va de 0,01 à 15 % en poids par rapport au poids total de la composition.

3. Composition selon la revendication 1 ou 2, **caractérisée en ce que** les particules de copolymère siliconé bloc présentent une taille moyenne en nombre inférieure ou égale à 2 microns.

4. Composition selon l'une quelconque des revendications précédentes, **caractérisée en ce que** l'organopolysiloxane (i) est choisi parmi les composés de formule (I) : 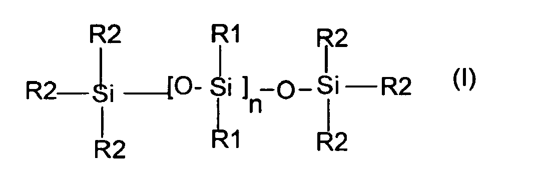 dans laquelle R₁ et R₂ indépendamment les uns des autres représentent un groupe hydrocarboné ayant de 1 à 20 atomes de carbone ou un groupe aryle ou un groupe réactif, n est un nombre entier supérieur à 1, à la condition qu'il y ait en moyenne entre un et deux groupes réactifs par polymère.

5. Composition selon la revendication précédente, **caractérisée en ce que** le groupe réactif est choisi parmi l'hydrogène ; les groupes aliphatiquement insaturés ; le groupe hydroxyle ; les groupes alcoxy ; les groupes alcoxy-alcoxy ; le groupe acétoxy ; les groupes aminés ; et leurs mélanges.

6. Composition selon la revendication 4 ou 5, **caractérisée en ce que** R₁ représente un groupe méthyle.

7. Composition selon l'une quelconque des revendications 4 à 6, **caractérisée en ce que** R₂ en bout de chaîne représente un groupe vinyle.

8. Composition selon l'une quelconque des revendications précédentes, **caractérisée en ce que** le composé organosiliconé (ii) est choisi parmi les polysiloxanes de formule (1) ou les composés agissant comme agent d'extension de chaîne.

9. Composition selon la revendication précédente, **caractérisée en ce que** le composé (ii) est un organohydrogenopolysiloxane liquide de formule (II) : 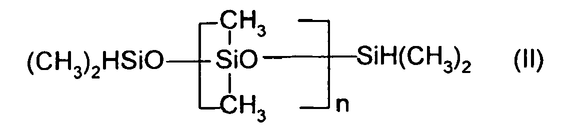 où n est un nombre entier supérieur à 1 et de préférence supérieur à 10.

10. Composition selon la revendication précédente, **caractérisée en ce que**, dans la formule (II), n est égal à 20.

11. Composition selon l'une quelconque des revendications précédentes, **caractérisée en ce que** la dispersion aqueuse de particules de copolymère siliconé est obtenue par mélange d'eau, d'au moins un émulsifiant, du polysiloxane (i), du composé organosiliconé (ii) et d'un catalyseur.

12. Composition selon la revendication précédente, **caractérisée en ce que** la dispersion est une dispersion aqueuse de copolymère divinyldimethicone / dimethicone, C₁₂-C₁₃ Pareth-3 et C₁₂-C₁₃ Pareth-23.

13. Composition selon l'une quelconque des revendications précédentes, **caractérisée en ce que** l'organopolysiloxane élastomère est obtenu par réaction d'addition et de réticulation, en présence d'un catalyseur, d'au moins :
- un premier organopolysiloxane (iii) ayant deux groupements vinyliques en position α-ω de la chaîne siliconée par molécule ; et
- un second organopolysiloxane (iv) ayant au moins un atome d'hydrogène lié à un atome de silicium par molécule.

14. Composition selon la revendication précédente, **caractérisée en ce que** le premier organopolysiloxane (iii) est un α-ω-diméthylvinyl polydiméthylsiloxane.

15. Composition selon la revendication 13 ou 14, **caractérisée en ce que** l'organopolysiloxane est sous forme d'un gel obtenu selon les étapes suivantes :
- (a) mélange du premier et second organopolysiloxanes (iii) et (iv) ;
- (b) ajout d'une phase huileuse au mélange de l'étape (a) ;
- (c) polymérisation du premier et second organopolysiloxanes (iii) et (iv) en phase huileuse en présence d'un catalyseur de platine.

16. Composition selon l'une des revendications précédentes, **caractérisée en ce que** l'organopolysiloxane élastomère se présente sous forme d'un gel anhydre.

17. Composition selon l'une des revendications précédentes, **caractérisée en ce que** la quantité l'organopolysiloxane élastomère va de 2 à 20 % en poids par rapport au poids total de la composition, et de préférence de 2 à 10 % en poids par rapport au poids total de la composition.

18. Composition selon l'une quelconque des revendications précédentes, **caractérisée en ce que** la charge est choisie parmi les charges organiques, les charges minérales et leurs mélanges.

19. Composition selon l'une quelconque des revendications précédentes, **caractérisée en ce que** la charge est une charge organique choisie parmi les particules de polyamide ; les poudres et billes de polyéthylène ; les microsphères à base de copolymères acryliques ou méthacryliques ; les microsphères de polyméthacrylate de méthyle ; les poudres de copolymère éthylène-acrylate ; les poudres expansées ; les poudres de matériaux organiques naturels ; les microbilles de résine de silicone ; et leurs mélanges.

20. Composition selon l'une quelconque des revendications précédentes, **caractérisée en ce que** la charge est une charge minérale choisie parmi le talc ; le kaolin ; le nitrure de bore ; les oxydes métalliques ; les micas ; les nacres ; les poudres de silice ; l'oxychlorure de bismuth ; le stéarate de zinc ; les particules de sel de métal alcalin ou alcalinoterreux ; les particules de platine ; les alumines ; les alumino-silicates ; les silicates mixtes de métaux alcalins et/ou alcalino-terreux ; les zéolithes ; la magnésie ; les matériaux composites à base de charges minérales ; et leurs mélanges.

21. Composition selon l'une quelconque des revendications précédentes, **caractérisée en ce que** la quantité de charge(s) va de 0,01 à 30 % en poids, et de préférence de 0,05 à 20 % en poids par rapport au poids total de la composition.

22. Composition selon l'une quelconque des revendications précédentes, **caractérisée en ce que** l'alcool primaire en C₂-C₃ est l'éthanol.

23. Composition selon l'une quelconque des revendications précédentes, **caractérisée en ce que** l'alcool primaire en C₂-C₃ est présent en une quantité allant de 2 à 20 % en poids, de préférence de 3 à 10 % en poids par rapport au poids total de la composition.

24. Composition selon l'une quelconque des revendications précédentes, **caractérisée en ce qu**'elle comprend au moins un actif choisi parmi les agents tenseurs, les sels d'alpha-hydroxyacides, les caroténoïdes, les extraits végétaux, le hyaluronate de sodium, les dérivés de vitamine C, et leurs mélanges.

25. Composition selon la revendication précédente, **caractérisée en ce que** l'actif est choisi parmi les polymères siliconés greffés, les gluconates d'alpha-hydroxyacides, le lycopène, l'ascorbyl-2 glucoside, l'ascorbyl phosphate de magnésium, le hyaluronate de sodium ; l'extrait d'Ulmaire ; et leurs mélanges.

26. Composition selon l'une quelconque des revendications précédentes, **caractérisée en ce qu**'elle se présente sous forme d'une émulsion huile-dans-eau.

27. Composition selon l'une quelconque des revendications précédentes, **caractérisée en ce qu**'elle constitue un produit de soin, de protection et /ou de maquillage de la peau.

28. Utilisation cosmétique d'une composition cosmétique selon l'une quelconque des revendications 1 à 26, pour donner un aspect mat à la peau et/ou pour estomper les défauts du relief de la peau tels que les microreliefs, les rides, les pores tout en conférant à celle-ci un aspect naturel.

29. Procédé cosmétique pour donner un aspect mat à la peau et/ou pour estomper les défauts du relief de la peau, consistant à appliquer sur la peau, une composition cosmétique selon l'une quelconque des revendications 1 à 26.

## Claims

1. Composition for topical application, comprising at least one elastomeric solid organopolysiloxane, at least one filler, at least one C₂-C₃ primary alcohol and an aqueous dispersion of at least one substantially linear block silicone copolymer that is non-crosslinked and free of oxyalkylene group, the said copolymer being obtained by chain-extension reaction, in the presence of a catalyst, starting with at least:
- (a) one polysiloxane (i) having at least one reactive group and preferably one or two reactive groups per molecule; and
- (b) one organosilicon compound (ii) that reacts with the polysiloxane (i) by chain-extension reaction.

2. Composition according to Claim 1, **characterized in that** the amount of block silicone copolymer ranges from 0.01% to 15% by weight relative to the total weight of the composition.

3. Composition according to Claim 1 or 2, **characterized in that** the block silicone copolymer particles have a number-average size of less than or equal to 2 microns.

4. Composition according to any one of the preceding claims, **characterized in that** the organopolysiloxane (i) is chosen from the compounds of formula (I): 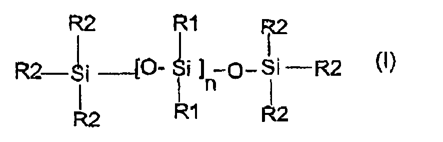 in which R₁ and R₂, independently of each other, represent a hydrocarbon-based group containing from 1 to 20 carbon atoms or an aryl group or a reactive group, n is an integer greater than 1, with the proviso that there are on average between one and two reactive groups per polymer.

5. Composition according to the preceding claim, **characterized in that** the reactive group is chosen from hydrogen ; aliphatically unsaturated groups; a hydroxyl group; alkoxy groups; alkoxyalkoxy groups; an acetoxy group; amino groups; and mixtures thereof.

6. Composition according to Claim 4 or 5, **characterized in that** R₁ represents a methyl group.

7. Composition according to any one of Claims 4 to 6, **characterized in that** R₂ at the end of a chain represents a vinyl group.

8. Composition according to any one of the preceding claims, **characterized in that** the organosilicon compound (ii) is chosen from the polysiloxanes of formula (I) or compounds acting as chain extenders.

9. Composition according to the preceding claim, **characterized in that** compound (ii) is a liquid organohydrogenopolysiloxane of formula (II): 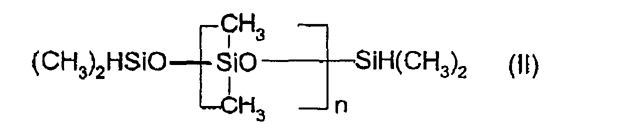 in which n is an integer greater than 1 and preferably greater than 10.

10. Composition according to the preceding claim, **characterized in that**, in formula (II), n is equal to 20.

11. Composition according to any one of the preceding claims, **characterized in that** the aqueous dispersion of silicone copolymer particles is obtained by mixing water, at least one emulsifier, the polysiloxane (i), the organosilicon compound (ii) and a catalyst.

12. Composition according to the preceding claim, **characterized in that** the dispersion is an aqueous dispersion of divinyl dimethicone/dimethicone copolymer, C₁₂-C₁₃ Pareth-3 and C₁₂-C₁₃ Pareth-23.

13. Composition according to any one of the preceding claims, **characterized in that** the elastomeric organopolysiloxane is obtained by addition reaction and crosslinking reaction, in the presence of a catalyst, of at least:
- one first organopolysiloxane (iii) containing two vinylic groups in the α-ω position of the silicone chain per molecule; and
- one second organopolysiloxane (iv) containing at least one hydrogen atom linked to a silicon atom per molecule.

14. Composition according to the preceding claim, **characterized in that** the first organopolysiloxane (iii) is an α-ω-dimethylvinyl polydimethylsiloxane.

15. Composition according to Claim 13 or 14, **characterized in that** the organopolysiloxane is in the form of a gel obtained according to the following steps:
- (a) mixing of the first and second organopolysiloxanes (iii) and (iv);
- (b) addition of an oily phase to the mixture from step (a);
- (c) polymerization of the first and second organopolysiloxanes (iii) and (iv) in oily phase in the presence of a platinum catalyst.

16. Composition according to one of the preceding claims, **characterized in that** the elastomeric organopolysiloxane is in the form of an anhydrous gel.

17. Composition according to one of the preceding claims, **characterized in that** the amount of elastomeric organopolysiloxane ranges from 2% to 20% by weight relative to the total weight of the composition, and preferably from 2% to 10% by weight relative to the total weight of the composition.

18. Composition according to any one of the preceding claims, **characterized in that** the filler is chosen from organic fillers, mineral fillers, and mixtures thereof.

19. Composition according to any one of the preceding claims, **characterized in that** the filler is an organic filler chosen from polyamide particles; polyethylene powders and beads; microspheres based on acrylic or methacrylic copolymers; polymethyl methacrylate microspheres; ethylene-acrylate copolymer powders; expanded powders; powders of natural organic materials; silicone resin microbeads; and mixtures thereof.

20. Composition according to any one of the preceding claims, **characterized in that** the filler is a mineral filler chosen from talc; kaolin; boron nitride; metal oxides; micas; nacres; silica powders; bismuth oxychloride: zinc stearate; alkali metal or alkaline-earth metal salt particles; platinum particles; aluminas; aluminosilicates; mixed silicates of alkali metals and/or alkaline-earth metals; zeolites; magnesia; composite materials based on mineral fillers; and mixtures thereof.

21. Composition according to any one of the preceding claims, **characterized in that** the amount of filler(s) ranges from 0.01% to 30% by weight and preferably from 0.05% to 20% by weight relative to the total weight of the composition.

22. Composition according to any one of the preceding claims, **characterized in that** the C₂-C₃ primary alcohol is ethanol.

23. Composition according to any one of the preceding claims, **characterized in that** the C₂-C₃ primary alcohol is present in an amount ranging from 2% to 20% by weight and preferably from 3% to 10% by weight relative to the total weight of the composition.

24. Composition according to any one of the preceding claims, **characterized in that** it comprises at least one active agent chosen from tensioning agents, α-hydroxy acid salts, carotenoids, plant extracts, sodium hyaluronate and vitamin C derivatives, and mixtures thereof.

25. Composition according to the preceding claim, **characterized in that** the active agent is chosen from grafted silicone polymers, α-hydroxy acid gluconates, lycopene, 2-ascorbylglucoside, magnesium ascorbylphosphate, sodium hyaluronate; extract of meadow-sweet; and mixtures thereof.

26. Composition according to any one of the preceding claims, **characterized in that** it is in the form of an oil-in-water emulsion.

27. Composition according to any one of the preceding claims, **characterized in that** it constitutes a care product, a protective product and/or a makeup product for the skin.

28. Cosmetic use of a cosmetic composition according to any one of Claims 1 to 26, to give the skin a matt appearance and/or to fade out skin relief defects such as microreliefs, wrinkles and pores, while at the same time making the skin look natural.

29. Cosmetic process to give the skin a matt appearance and/or to fade out skin relief defects, which consists in applying to the skin a cosmetic composition according to any one of Claims 1 to 26.

## Patentansprüche

1. Zusammensetzung für die topische Anwendung, die mindestens ein festes elastomeres Organopolysiloxan, mindestens einen Füllstoff, mindestens einen primären C₂₋₃-Alkohol und eine wässrige Dispersion mindestens eines Siliconblockcopolymers enthält, das im Wesentlichen linear und nicht vernetzt ist und keine Oxyalkylengruppe enthält und durch Kettenverlängerung in Gegenwart eines Katalysators erhalten wird ausgehend von zumindest:
- (a) einem Polysiloxan (i), das mindestens eine reaktive Gruppe und vorzugsweise eine oder zwei reaktive Gruppen pro Molekül aufweist; und
- (b) einer Organosiliconverbindung (ii), die mit dem Polysiloxan (i) durch Kettenverlängerung reagiert.

2. Zusammensetzung nach Anspruch 1, **dadurch gekennzeichnet, dass** der Mengenanteil des Siliconblockcopolymers im Bereich von 0,01 bis 15 Gew.-%, bezogen auf das Gesamtgewicht der Zusammensetzung, liegt.

3. Zusammensetzung nach Anspruch 1 oder 2, **dadurch gekennzeichnet, dass** die Partikel des Siliconblockcopolymers eine zahlenmittlere Größe von 2 µm oder darunter aufweisen.

4. Zusammensetzung nach einem der vorhergehenden Ansprüche, **dadurch gekennzeichnet, dass** das Organopolysiloxan (i) unter den Verbindungen der folgenden Formel (I) ausgewählt ist: 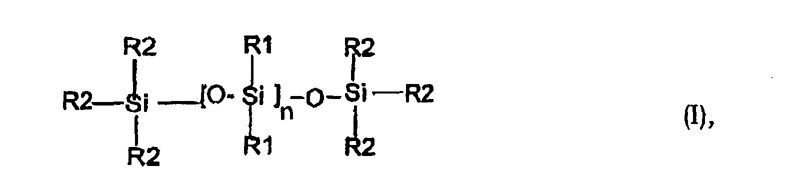 worin R₁ und R₂ unabhängig voneinander eine Kohlenwasserstoffgruppe mit 1 bis 20 Kohlenstoffatomen oder eine Arylgruppe oder eine reaktive Gruppe bedeuten und n eine ganze Zahl über 1, ist, mit der Maßgabe, dass im Mittel eine bis zwei reaktive Gruppen pro Polymer vorhanden sind.

5. Zusammensetzung nach dem vorhergehenden Anspruch, **dadurch gekennzeichnet, dass** die reaktive Gruppe ausgewählt ist unter Wasserstoff; ungesättigten aliphatischen Gruppen; der Hydroxygruppe; Alkoxygruppen; Alkoxyalkoxygruppen; der Acetoxygruppe; aminierten Gruppen; und deren Gemischen.

6. Zusammensetzung nach Anspruch 4 oder 5, **dadurch gekennzeichnet, dass** R₁ eine Methylgruppe bedeutet.

7. Zusammensetzung nach einem der Ansprüche 4 bis 6, **dadurch gekennzeichnet, dass** die Gruppe R₂ am Kettenende eine Vinylgruppe ist.

8. Zusammensetzung nach einem der vorhergehenden Ansprüche, **dadurch gekennzeichnet, dass** das Organosilicon (ii) unter den Polysiloxanen der Formel (I) oder Verbindungen, die als Kettenverlängerer wirken, ausgewählt ist.

9. Zusammensetzung nach dem vorhergehenden Anspruch, **dadurch gekennzeichnet, dass** die Verbindung (ii) ein flüssiges Organohydrogenopolysiloxan der Formel (II) ist: 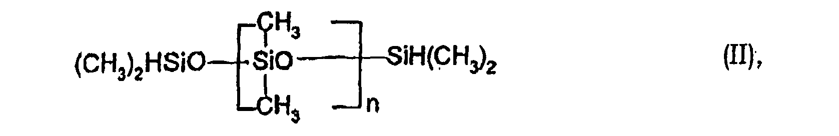 worin n eine ganze Zahl über 1 und vorzugsweise über 10 bedeutet.

10. Zusammensetzung nach dem vorhergehenden Anspruch, **dadurch gekennzeichnet, dass** n in der Formel (II) 20 bedeutet.

11. Zusammensetzung nach einem der vorhergehenden Ansprüche, **dadurch gekennzeichnet, dass** die wässrige Dispersion der Partikel des Siliconcopolymers durch Mischen von Wasser, mindestens eines Emulgators, des Polysiloxans (i), des Organosilicons (ii) und eines Katalysators erhalten wird.

12. Zusammensetzung nach dem vorhergehenden Anspruch, **dadurch gekennzeichnet, dass** die Dispersion eine wässrige Dispersion des Copolymers Divinyldimethicon/Dimethicon, von C₁₂-C₁₃ Pareth-3 und von C₁₂-C₁₃ Pareth-23 ist.

13. Zusammensetzung nach einem der vorhergehenden Ansprüche, **dadurch gekennzeichnet, dass** das elastomere Organopolysiloxan in Gegenwart eines Katalysators durch Addition und Vernetzung von zumindest den folgenden Verbindungen hergestellt wird:
- eines ersten Organopolysiloxans (iii), das zwei Vinylgruppen in α-ω-Stellung der Siliconkette pro Molekül aufweist; und
- eines zweiten Organopolysiloxans (iv), das mindestens ein an ein Siliciumatom gebundenes Wasserstoffatom pro Molekül besitzt.

14. Zusammensetzung nach dem vorhergehenden Anspruch, **dadurch gekennzeichnet, dass** das erste Organopolysiloxan (iii) ein α-ω-Dimethylvinylpolydimethylsiloxan ist.

15. Zusammensetzung nach Anspruch 13 oder 14, **dadurch gekennzeichnet, dass** das Organopolysiloxan in Form eines Gels vorliegt, das gemäß den folgenden Schritten erhalten wird:
- (a) Mischen des ersten und des zweiten Organopolysiloxans (iii) und (iv);
- (b) Zugabe einer Ölphase zu dem Gemisch aus Schritt (a);
- (c) Polymerisation des ersten und zweiten Organopolysiloxans (iii) und (iv) in der Ölphase in Gegenwart eines Platinkatalysators.

16. Zusammensetzung nach einem der vorhergehenden Ansprüche, **dadurch gekennzeichnet, dass** das elastomere Organopolysiloxan in Form eines wasserfreien Gels vorliegt.

17. Zusammensetzung nach einem der vorhergehenden Ansprüche, **dadurch gekennzeichnet, dass** der Mengenanteil des elastomeren Organopolysiloxans im Bereich von 2 bis 20 Gew.-%, bezogen auf das Gesamtgewicht der Zusammensetzung, und vorzugsweise 2 bis 10 Gew.-%, bezogen auf das Gesamtgewicht der Zusammensetzung, liegt.

18. Zusammensetzung nach einem der vorhergehenden Ansprüche, **dadurch gekennzeichnet, dass** der Füllstoff unter den organischen Füllstoffen, anorganischen Füllstoffen und deren Gemischen ausgewählt ist.

19. Zusammensetzung nach einem der vorhergehenden Ansprüche, **dadurch gekennzeichnet, dass** der Füllstoff ein organischer Füllstoff ist, der unter den Polyamidpartikeln, Pulvern und Kugeln aus Polyethylen; Mikrosphären auf der Basis von Acryl- oder Methacrylcopolymeren; Mikrosphären von Polymethylmethacrylat; Pulvern aus Ethylen-Acrylat-Copolymer; expandierten Pulvern; Pulvern natürlicher organischer Stoffe; Mikrokugeln aus Siliconharz; und deren Gemischen ausgewählt ist.

20. Zusammensetzung nach einem der vorhergehenden Ansprüche, **dadurch gekennzeichnet, dass** der Füllstoff ein anorganischer Füllstoff ist, der ausgewählt ist unter Talk; Kaolin; Bornitrid; Metalloxiden; Glimmern; Perlglanzstoffen; Kieselsäurepulvern; Bismutoxidchlorid; Zinkstearat; Partikeln eines Alkalimetall- oder Erdalkalimetallsalzes; Platinpartikeln; Aluminiumoxiden; Aluminosilicaten; gemischten Silicaten von Alkali- und/oder Erdalkalimetallen; Zeolithen; Magnesiumoxid; Verbundwerkstoffen auf der Basis von anorganischen Füllstoffen und deren Gemischen.

21. Zusammensetzung nach einem der vorhergehenden Ansprüche, **dadurch gekennzeichnet, dass** der Mengenanteil des Füllstoffs oder der Füllstoffe im Bereich von 0,01 bis 30 Gew.-% und vorzugsweise 0,05 bis 20 Gew.-%, bezogen auf das Gesamtgewicht der Zusammensetzung, liegt.

22. Zusammensetzung nach einem der vorhergehenden Ansprüche, **dadurch gekennzeichnet, dass** es sich bei dem primären C₂₋₃-Alkohol um Ethanol handelt.

23. Zusammensetzung nach einem der vorhergehenden Ansprüche, **dadurch gekennzeichnet, dass** der primäre C₂-₃-Alkohol in einer Menge von 2 bis 20 Gew.-% und vorzugsweise 3 bis 10 Gew,-%, bezogen auf das Gesamtgewicht der Zusammensetzung, enthalten ist.

24. Zusammensetzung nach einem der vorhergehenden Ansprüche, **dadurch gekennzeichnet, dass** sie mindestens einen Wirkstoff enthält, der unter den straffenden Wirkstoffen, Salzen von alpha-Hydroxysäuren, Carotinoiden, Pflanzenextrakten, Natriumhyaluronat, Derivaten von Vitamin C und deren Gemischen ausgewählt ist.

25. Zusammensetzung nach dem vorhergehenden Anspruch, **dadurch gekennzeichnet, dass** der Wirkstoff unter den gepfropften Siliconpolymeren, Gluconaten von alpha-Hydroxysäuren, Lycopin, 2-Ascorbylglucosid, Natriumascorbylphosphat, Natriumhyaluronat; Mädesüßextrakt und deren Gemischen ausgewählt ist.

26. Zusammensetzung nach einem der vorhergehenden Ansprüche, **dadurch gekennzeichnet, dass** sie in Form einer Öl-in-Wasser-Emulsion vorliegt.

27. Zusammensetzung nach einem der vorhergehenden Ansprüche, **dadurch gekennzeichnet, dass** sie ein Produkt für die Pflege, zum Schutz und/oder zum Schminken der Haut darstellt.

28. Kosmetische Verwendung einer kosmetischen Zusammensetzung nach einem der Ansprüche 1 bis 26, um der Haut ein mattes Aussehen zu geben und/oder die Mängel des Hautreliefs wie Mikroreliefe, Falten und Poren zu kaschieren und ihr gleichzeitig ein natürliches Aussehen zu geben.

29. Kosmetisches Verfahren, um die Haut matt aussehen zu lassen und/oder Mängel des Hautreliefs zu kaschieren, das darin besteht, auf die Haut eine kosmetische Zusammensetzung nach einem der Ansprüche 1 bis 26 aufzutragen.
